# EUROPÄISCHE PATENTSCHRIFT

(11) **EP 0 831 673 B1**
(45) Veröffentlichungstag und Bekanntmachung des Hinweises auf die Patenterteilung: **06.08.2008**
(21) Anmeldenummer: 97114603.0
(22) Anmeldetag: 22.08.1997
(51) Int. Cl.: H04R 25/00

(54) **Implantierbares Mikrofon**
Implantable microphone
Microphone implantable

(30) Priorität: 18.09.1996 DE 19638158
(43) Veröffentlichungstag der Anmeldung: 25.03.1998
(73) Patentinhaber: PHONAK AG, 8712 Stäfa (CH)
(72) Erfinder: Müller, Gerd, Dr. Dipl.-Phys., 85716 Unterschleissheim (DE); Leysieffer, Hans, Dr. Dipl.-Ing., 82024 Taufkirchen (DE)
(74) Vertreter: Schwan, Gerhard

(56) Entgegenhaltungen:
- WO-A-92/22107
- DE-A1- 3 741 180
- DE-A1- 3 918 329
- US-A- 3 557 775

## Beschreibung

Die Rehabilitation sensorischer Hörstörungen mit teilimplantierbaren, elektronischen Systemen hat in den letzten zehn Jahren einen bedeutenden Stellenwert erhalten. Insbesondere gilt dies für den Patientenkreis, bei dem das Gehör durch Unfall, Krankheit oder sonstige Einflüsse vollständig ausgefallen oder bereits von Geburt an nicht funktionsfähig ist. Ist in diesen Fällen nur das Innenohr (Cochlea) und nicht die nach zentral führende neuronale Hörbahn betroffen, kann mit elektrischen Reizsignalen der verbliebene Hörnerv stimuliert und somit ein Höreindruck erzeugt werden, der bis zu einem offenen Sprachverständnis führen kann. Bei diesen sogenannten Cochlea Implantaten wird ein Reizelektroden-Array in die Cochlea eingeführt, das von einem elektronischen System angesteuert wird, wobei dieses hermetisch dicht und biokompatibel eingekapselte Elektronikmodul operativ im knöchernen Bereich hinter dem Ohr (Mastoid) eingebettet ist. Das elektronische System enthält jedoch im wesentlichen nur Dekodier- und Treiberschaltungen für die Reizelektroden; die akustische Schallaufnahme, die Wandlung dieses Schallsignals in elektrische Signale und deren weitere Verarbeitung erfolgt grundsätzlich extern in einem sogenannten Sprachprozessor, der außen am Körper getragen wird. Der Sprachprozessor setzt die vorverarbeiteten Signale entsprechend kodiert auf ein hochfrequentes Trägersignal um, das über eine induktive Kopplung durch die geschlossene Haut (transkutan) zu dem Implantat übertragen wird. Das schallaufnehmende Mikrofon befindet sich ausnahmslos außerhalb des Körpers und in den meisten Anwendungen in einem an der Ohrmuschel getragenen Gehäuse eines Hinter-dem-Ohr-Hörgerätes (HdO) und ist mit einem Kabel mit dem Sprachprozessor verbunden. Solche Cochlea Implantate und deren Komponenten sind beispielhaft in folgenden Patentschriften beschrieben: EP-A-0 572 382 (Daly), WO-A-92/22107 (Kuzma), AU 624989 (Seligman und Dowell), US-A-4 532 930 (Crosby et al.) und EP-A-0 076 070 (Hochmair).

Neben der Rehabilitation gehörloser bzw. ertaubter Patienten mit Cochlea Implantaten existieren seit geraumer Zeit Ansätze, Patienten mit einer sensorineuralen Hörstörung, die operativ nicht behebbar ist, mit teil- bzw. vollimplantierbaren Hörgeräten eine bessere Rehabilitation als mit konventionellen Hörgeräten zu bieten. Das Prinzip besteht in den überwiegenden Ausführungsformen darin, ein Ossikel des Mittelohres oder das Innenohr direkt über einen mechanischen bzw. hydromechanischen Reiz zu stimulieren und nicht über das verstärkte akustische Signal eines konventionellen Hörgerätes, bei dem das verstärkte Schallsignal dem äußeren Gehörgang zugeführt wird. Der aktorische Stimulus dieser elektromechanischen Systeme wird mit verschiedenen physikalischen Wandlerprinzipien realisiert wie z.B. durch elektromagnetische und piezoelektrische Systeme. Der Vorteil dieser Verfahren wird hauptsächlich in der gegenüber konventionellen Hörgeräten verbesserten Klangqualität und bei vollimplantierten Systemen in der Unsichtbarkeit der Hörprothese gesehen. Solche teil- und vollimplantierbaren elektromechanischen Hörgeräte sind beispielhaft von Yanigahara und Suzuki et al. (Arch Otolaryngol Head Neck, Surg-Vol 113, 1987, pp. 869-872; Hoke, M. (ed), Advances in Audiology, Vol. 4, Karger Basel, 1988) und in zahlreichen Patentveröffentlichungen beschrieben: EP-A-0 499 940 (Leysief fer et al.), US-A-5 411 467 und EP-A-0 400 630 (Hortmann und Leysieffer), US-A-3 764 748 (Branch et al.), US-A-4 352 960 (Dormer et al.), US-A-5 015 225 (Hough et al.), US-A-5 015 224 (Maniglia), US-A-3 557 775 (Mahoney), US-A-3 712 962 (Eply), US-A-4 988 333 (Engebretson et al.), EP-A-0 263 254 (Schaefer), WO-A-92/09181 (Perkins) und WO-A-93/06666 (Buchele).

Sowohl bei Cochlea Implantaten wie auch den elektromechanischen Hörgeräten erscheint es heute als sehr sinnvoll, die Systeme so auszulegen, daß sie vollständig implantiert werden können. Neben der Notwendigkeit einen hohen mikroelektronischen Integrationsdichte der Signalverarbeitung und einer implantierbaren Energieversorgung spielen hierbei die Schallaufnahme und die Wandlung des akustischen Signals in ein elektrisches Signal eine ganz wesentliche Rolle. Es ist anzustreben, diesen Wandlungsvorgang so auszulegen, daß tatsächlich ein vollständig implantierbares System realisiert wird. Dies bedeutet, daß die akustische Signalaufnahme transkutan, das heißt, durch die geschlossene Haut erfolgen sollte. Dabei ist weiterhin zu berücksichtigen, daß die Wahl des Ortes dieser Schallwandlung audiologisch optimiert sein sollte, um eine weitgehende natürliche Störschallunterdrückung zu erhalten. Unter diesem wichtigen Aspekt bieten sich als sinnvoller Plazierungsort des schallwandelnden Elementes das Trommelfell selbst, der tiefliegende Bereich des äußeren Gehörgangs und auch die Ossikelkette im Mittelohrbereich in der Paukenhöhle an. Einige Patentschriften und Literaturstellen beschreiben die Realisierung solcher vollständig implantierbarer akusto-elektrischer Wandlersysteme; zur Beschreibung des Standes der Technik wird im folgenden auf diese Publikationen eingegangen.

Schaefer (US-A-4 729 366, US-A-4 850 962 und EP-A-0 263 254) beschreibt ein implantierbares Hörgerät, bei dem nach Entfernen des Amboß der Ossikelkette ein zweifaches Wandlersystem eingesetzt wird, wobei ein Element als mechano-elektrischer Wandler (Sensor: Mikrofon-Funktion) und das andere Element als elektromechanischer Wandler zur vibratorischen Stimulation des Steigbügels (Aktor-Funktion) dienen. Das Sensorelement ist mit dem verbleibenden Hammer über ein Koppelelement, das als mechanisch steifer Draht ausgeführt ist, verbunden und nimmt so die mechanischen Schwingungen des Trommelfells auf, die aus einer auf das Trommelfell einfallenden akustischen Welle resultieren. Das dem mechanischen Schwingungssignal entsprechende elektrische Signal des Sensorelementes wird einer verstärkenden Signalverarbeitungseinheit zugeführt, die wiederum das Aktorelement ansteuert, das über ein ebenfalls mechanisch steifes, drahtförmiges Koppelelement den Steigbügel in mechanische Schwingungen versetzt. Bei der Sensorankopplung sind verschiedene Varianten des Koppelelementes an den Hammergriff, den Hammerkopf und den Körper des Hammers dargestellt. Schaefer gibt mehrere mögliche physikalische Wandlerprinzipien für beide aktive Elemente an, darunter piezokeramische Wandler, Piezofilm-Wandler und elektromagnetische Systeme. Problematisch erscheinen bei der beschriebenen Mikrofonfunktions-Realisierung folgende Aspekte:
- Bei Verwendung eines piezokeramischen Wandlerelementes, das beispielhaft als stabförmiger Bimorph-Biegeschwinger ausgeführt ist, liegt die mechanische Eingangsimpedanz deutlich über der biologischen mechanischen Quellimpedanz, die in diesem Fall hauptsächlich von dem System Trommelfell mit dem damit fest verwachsenen Hammer gebildet wird. Daraus resultiert bei gegebenem Eingangsschalldruckpegel im äußeren Gehörgang eine sehr kleine Auslenkung des Piezoelementes, was wiederum bei gegebenem mechano-elektrischen Wandlungsfaktor des Piezoelementes zu sehr kleinen elektrischen Ausgangssignalen führt. Die daher notwendige hohe elektrische Verstärkung in dem signalverarbeitenden Modul reduziert bei heute gegebener, mikroelektronischer Verstärkertechnologie die nutzbare Dynamik des Gesamtsystem aufgrund eines unzureichenden Signal-Stör-Abstandes.
- Auch wenn oben genannter Nachteil durch Verwendung eines Wandlersystems mit deutlich reduziertem mechanischem Impedanzniveau wie z.B. dünne Piezofilme (z.B. PFDV, Polyvinylidenfluorid) abgeschwächt wird, bleibt ein weiterer Nachteil dieser Variante einer Mikrofon-Funktion bestehen: aufgrund heute bekannter komplexer Resonanzeigenschaften des Mittelohrapparates einschließlich des Trommelfells (z.B. "Analysis of dynamic behavior of human middle ear using a finite-element method", Wada, H. et al., J. Acoust. Soc. Am. 92 (6), Dec. 1992, pp. 3157 - 3168) kann nicht davon ausgegangen werden, daß bei angenommenem frequenzunabhängigem Eingangsschalldruck ein ebenfalls über der Frequenz ebener Schalldruckübertragungsfaktor erzielt wird. Daraus resultieren lineare Verzerrungen der Mikrofon-Übertragungsfunktion, die für die angestrebte Hörgeräte-Funktion unerwünscht und darüber hinaus inter-individuellen Schwankungen aufgrund unterschiedlicher Anatomie unterworfen sind.
- Die nicht näher ausgeführte technische Realisierung der notwendigen hermetisch dichten und biokompatiblen Kapselung des Sensorelementes erscheint insbesondere auch deshalb sehr schwierig, weil die mechanischen Schwingungen des Koppeldrahtes, der den Hammer mit dem Wandlerelement verbindet, durch die zwingend hermetisch dichte Wandung des Implantatgehäuses, das das aktive Wandlerelement enthält, geleitet werden müssen.

Engebretson et al. (US-A-4 988 333) beschreiben ein implantierbares akustisches Kuppler-System, bei dem eine mit einer beispielhaft aus Silikon bestehenden dünnen, kreisförmigen Membran abgeschlossene Kammer als schallaufnehmendes Element ausgebildet ist. Beispielhaft kann diese Membran über einen dünnen Koppeldraht, der im Mittelpunkt der Membran befestigt ist, mechanisch fest mit dem Hammer der Ossikelkette verbunden werden, wodurch die Trommelfellschwingungen als akustisches Signal innerhalb der Kammer abgebildet werden. Über einen Schalleitungsschlauch, der an diese aufnehmende Kuppelkammer angeschlossen ist, wird das Schallsignal zu einem Mikrofon geleitet, das aus Platzgründen nicht in der Paukenhöhle untergebracht ist, sondern in einem Implantat-Hauptgehäuse im Bereich des Antrums oder Mastoids. Dieses System wurde in einer miniaturisierten Ausführungsform (4,0 mm Durchmesser der Kuppelkammer) in seiner prinzipiellen Funktion in einem zweiwöchigen Tiermodell getestet (Deddens et al.: "Totally implantable hearing aids: the effects of skin thickness on microphone function", Am. J. Otolaryngol. 1990, 11, pp. 1-4), wobei die akustische Kuppelkammer mit einer 100 µm dicken Silikonscheibe als Schalleintrittsmembran abgeschlossen war.

Das grundlegende Prinzip einer Schallweiterleitung über ein Schlauchelement wird von weiteren Autoren beschrieben (Mahoney, US-A-3 346 704 und US-A-3 557 775, Nunley et al., US-A-3 882 285, Leysieffer et al., DE-A-39 40 632). Das eigentliche Mikrofonelement ist in allen beschriebenen Fällen in einem gekapselten Implantatgehäuse plaziert. Die Schalleinleitung zu diesem Mikrofon erfolgt über einen mechanisch starren oder flexiblen Schlauch, dessen Eingangsende beispielhaft mit einer dünnen Membran abgeschlossen ist, über die das einfallende Schallsignal aufgenommen wird. Der Ort der Schallaufnahme ist bei Mahoney der Bereich des Mastoids hinter dem Außenohr (Schlauchende mit Membran unter der geschlossenen Haut über dem Mastoid), bei Nunley et al. der Bereich des äußeren Gehörgangs, wobei der Zuleitungsschlauch von der dünnen Haut der Gehörgangswand bedeckt ist, und bei Leysieffer et al. die Paukenhöhle selbst, in die das mit einer dünnen Membran abgeschlossene Ende des Schallzuleitungsschlauches ragt.

Bei diesen Lösungen erscheinen folgende Aspekte als problematisch bzw. technisch schwierig zu realisieren:
- Neben dem eigentlichen Mikrofon sind zusätzliche volumenbehaftete Elemente notwendig, die die Implantierbarkeit bei den ohnehin sehr begrenzten Platzverhältnissen im Bereich des Mittelohres, Antrums und Mastoids erschweren und die unter Langzeitaspekten zu fordernde Biostabilität fraglich erscheinen lassen.
- Technisch ist unter heute allgemein bekannten Aspekten der Wasserdampfaufnahme von Silikonen im Volumenprozentbereich die Membran, die die Schlauchelemente oder Kuppelkammern schalleingangsseitig abschließt, die für die Langzeitstabilität zu fordernde hermetische Dichtheit des Implantates nicht zu realisieren, d.h., es besteht das wesentliche Risiko, daß einerseits die elektronischen Implantatkomponenten wie insbesondere das Mikrofon selbst durch eintretende Feuchtigkeit zerstört bzw. geschädigt werden und andererseits toxische Stoffe aus dem Implantat austreten und in das Körpergewebe eindringen können.
- Funktional bestehen ähnliche Einschränkungen des individuell reproduzierbaren, frequenzunabhängigen Übertragungsmaßes dieser Mikrofonsysteme wie bei dem System nach Schäfer (s.o.), da die dünnen Membranen und schalleitenden Schläuche lineare Verzerrungen bedingen können, die im Einzelfall mit aufwendigen elektronischen Maßnahmen kompensiert werden müssen.

Eine andere Variante wird von Suzuki und Yanigahara angegeben ("Middle Ear Implants: Implantable Hearing Aids", Hoke, M. (ed), Advances in Audiology, Vol. 4, Karger Basel, 1988). Ein zylinderförmiges Edelstahlgehäuse ist auf einer Seite mit einer kreisförmigen dünnen Edelstahlmembran hermetisch dicht abgeschlossen. Innerhalb dieses Gehäuses ist ein konventionelles Miniatur-Elektret-Mikrofon untergebracht, dessen Schalleintrittsöffnung in einen ebenfalls dichten Zwischenraum ragt, der von der Gehäusemembran und einer internen Zwischenwandung gebildet wird. Die drei elektrischen Mikrofonanschlüsse werden über eine dreipolige hermetische Signaldurchführung, die neben der Mikrofonkapsel plaziert ist, aus dem Edelstahlgehäuse herausgeführt und sind mit der sich anschließenden implantierbaren Mikrofonanschlußleitung verbunden. Das Gehäuseinnere ist aus Gründen des Korrosionsschutzes mit einem Edelgas (Argon) gefüllt. Das Gehäuse wird in der Mastoidhöhle so implantiert, daß die Gehäuseseite mit der kreisförmigen Membran in einer entsprechenden Bohrung in der hinteren Gehörgangswand des knöchernen Bereich des Gehörgangs plaziert wird und die Gehörgangshaut durch vollständige Bedeckung in direktem mechanischen Kontakt zu der dünnen Edelstahlmembran steht. Fällt eine Schallwelle in den Gehörgang, wird die Metallmembran mit der darüberliegenden Gehörgangshaut in mechanische Schwingungen versetzt, was zu einer Druckschwankung in dem kleinvolumigen Zwischenraum im Inneren des Gehäuses führt. Diese Druckschwankung wird von dem internen Mikrofon als entsprechendes akustisches Signal aufgenommen und in ein elektrisches Signal umgewandelt, das über die Anschlußleitung der weiterverarbeitenden Elektronik zugeführt werden kann. Hierbei ist das System so ausgelegt, daß die erste mechanische Resonanz der schwingfähigen Metallmembran spektral am oberen Ende des audiologischen Übertragungsbereiches liegt und das Schalldruckübertragungsmaß unterhalb dieser Resonanz frequenzunabhängig ist und somit keine linearen Verzerrungen auftreten. Die Gehörgangshaut stellt im wesentlichen einen additiven dynamischen Massenbelag für die schwingfähige Membran dar, so daß sich die Resonanzfrequenz bei Belag mit der Haut gegenüber dem Leerlauf ohne Hautbelag lediglich zu tieferen Frequenzen verschiebt, ohne daß sich das Übertragungsmaß und damit die Gesamtmikrofonempfindlichkeit verändert. Das Design ist so ausgelegt, daß die Resonanzfrequenz im implantierten Zustand, d.h. bei Belag mit der Gehörgangshaut, bei 3 bis 5 kHz liegt und somit zumindest grundsätzlich der audiologisch wichtige Frequenzbereich übertragen werden kann. Als Schalldruckübertragungsfaktor wird ca. 2 mV/Pa angegeben. Dieser Wert wird als ausreichend für eine elektronische Weiterverarbeitung mit akzeptablem Signal-Störabstand bezeichnet. Die Außenabmessungen dieses zylinderförmigen Mikrofonmoduls betragen 8,0 mm im Durchmesser und 4,0 mm in der Höhe.

Dieses System weist gegenüber den anderen hier angeführten Realisierungsformen eines implantierbaren Mikrofons folgende Vorteile auf:
- Aufgrund der ganzmetallischen Ausführung ist das Modul hermetisch dicht und somit geschützt gegen Körperflüssigkeiten.
- Die funktionale Gestaltung der schallaufnehmenden Membran ermöglicht einen weitgehend frequenzunabhängigen Schalldruckübertragungsfaktor bis zur ersten Resonanzfrequenz.
- Die Fehlanpassung des mechanischen Systems der Membran gegenüber der akustischen Impedanz der eintreffenden Schallwelle bleibt in einem akzeptablen Bereich, so daß ein technisch verwertbarer Übertragungsfaktor resultiert.

Dieses Modul weist jedoch aufgrund seiner äußeren Geometrie einen wesentlichen Nachteil auf: umfangreiche eigene klinische Ermittlungen haben ergeben, daß es unmöglich ist, ein zylinderförmiges Modul mit einem Gesamtdurchmesser von 8,0 mm so im knöchernen Bereich des Gehörgangs und somit in einer Bohrung in der Gehörgangswand zu plazieren, daß einerseits eine vollständige Bedeckung der kreisförmigen, planen Oberfläche mit Gehörgangshaut erfolgt und andererseits gesichert werden kann, daß dieses Modul unter Langzeitaspekten biostabil implantierbar ist. Dies liegt im wesentlichen daran, daß erstens der äußere Gehörgang einen Durchmesser von unter 10 mm aufweist und so eine kreisförmige Membran mit 8,0 mm Durchmesser nicht als berührende, tangentiale Fläche bezeichnet werden kann, sondern eher eine Schnittebene darstellt, und zweitens die Entnahme eines so großen knöchernen Bereiches in der Gehörgangswand die ausreichende Versorgung der über der Metallmembran verbleibenden Gehörgangshaut als äußerst fraglich erscheinen läßt. Insgesamt erscheint es als sehr zweifelhaft, ob die beschriebenen Mikrofonsysteme überhaupt im knöchernen Bereich des Gehörgangs implantierbar sind oder ob eine Plazierung lediglich im knorpeligen oder Weichteile-Bereich des Gehörgangs möglich ist. Im letzteren Fall besteht bekannterweise das hohe Risiko des Wanderns eines Implantates, da eine adäquate mechanisch feste und langzeitstabile Verankerung des Mikrofongehäuses in knöchernen Strukturen nicht möglich ist.

Daher ist es eine Aufgabe der vorliegenden Erfindung, ein implantierbares Mikrofon zu schaffen, welches sich sicher und langzeitstabil implantieren läßt.

Zur Lösung dieser Aufgabe beschreibt die vorliegende Erfindung eine neue Variante für ein vollständig implantierbares Mikrofon zur Verwendung in implantierbaren Hörhilfesystemen, die auf dem von Suzuki und Yanigahara beschriebenen Prinzip eines hermetisch dichten Membrangehäuses beruht, jedoch durch eine prinzipiell abgewandelte Gehäusegeometrie eine deutliche Reduzierung der Außenabmessungen unter Beibehaltung bzw. Verbesserung der funktionalen Betriebsparameter ermöglicht, wobei diese Geometrie den gegebenen, natürlichen anatomischen Bedingungen des Implantationsortes im Bereich der Mastoidhöhle und der hinteren Gehörgangswand angepaßt ist.

Insbesondere weist bei einem implantierbaren Mikrofon für eine der Anregung des Gehörs dienende implantierbare Hörhilfe mit einer Mikrofonkapsel, die in einem Gehäuse allseitig hermetisch dicht untergebracht ist, und mit einer elektrischen Durchführungsanordnung zum Durchführen mindestens eines elektrischen Anschlusses von dem Innenraum des Gehäuses zu dessen Außenseite, das Gehäuse erfindungsgemäß mindestens zwei Schenkel auf, deren Achsen in einem Winkel mit Bezug aufeinander ausgerichtet sind, wobei der eine Schenkel die Mikrofonkapsel aufnimmt und mit einer Schalleintrittsmembran versehen ist, und wobei der andere Schenkel gegenüber der Ebene der Schalleintrittsmembran zurückversetzt ist und die elektrische Durchführungsanordnung in einem Bereich aufnimmt, der außerhalb einer zur Schalleintrittsmembran senkrechten Projektion der Mikrofonkapsel liegt.

Unter audiologischen Gesichtspunkten ist als optimaler Schallaufnahmeort für ein implantierbares Mikrofon der tiefliegende Bereich des Gehörgangs anzusehen, da dort nahe dem biologisch natürlichen Ort des Trommelfells die akustischen Eigenschaften des Außenohres und des Gehörgangs zur richtungsabhängigen Ausfilterung von Störsignalen ausgenützt werden können. Die vorliegend erläuterte Gestaltung des Mikrofongehäuses gestattet es, den mit der Schalleintrittsmembran versehenen Gehäuseteil bezüglich der geometrischen Abmessungen so weit zu minimieren, daß ein Durchmesser der schallaufnehmenden Membran kleiner 5,0 mm erreicht wird. Detaillierte eigene klinische Untersuchungen haben gezeigt, daß mit diesem Membrandurchmesser noch eine nahezu plane Einpassung an den annähernd kreisför-migen Querschnitt des Gehörgangs im tieferliegenden knöchernen Bereich möglich ist. Dieser minimierte Durchmesser wird bestimmt von den Abmessungen des kleinsten heute verfügbaren Miniatur-Elektret-Mikrofons. Eine interne Plazierung der zusätzlich notwendi-gen elektronischen Bauelemente sowie der elektrischen Durchführungen zum Anschluß des Moduls neben der Mikrofonkapsel wie in der Ausführungsform von Suzuki und Yanigahara ist dabei nicht möglich. Das Mikrofongehäuse muß nach Mastoidektomie vom Mastoid aus in die hintere Gehörgangswand eingebrachte Bohrung geführt und dort so plaziert werden, daß ein mechanisch sicherer Kontakt der gesamten Membranoberfläche mit der über der eingebrachten Bohrung liegenden Gehörgangshaut erzielt wird. Der mit der Schalleintrittsmembran versehene, vorzugsweise zylinderförmige Bereich des Mikrofongehäuses hat daher zweckmäßig mindestens eine Länge, die dem statistischen Maximum der Dicke der hinteren Gehörgangswand entspricht. Als für das neu zu konstruierende Mikrofongehäuse wesentlich geometrie- und volumenbestimmend hat sich der Abstand des Sinus Sigmoideus, dessen Außenkonturen operativ aus klinischen Gründen nicht verändert werden dürfen, zu der hinteren Gehörgangswand herausgestellt. Dieser in manchen individuellen anatomischen Verhältnissen sehr kleine Abstand läßt konstruktiv eine gesamte axiale Anordnung von interner Mikrofonkapsel, elektronischen Bauelementen und der notwendigen hermetischen Signaldurchführung für den elektrischen Anschluß des Mikrofons nicht zu.

Daher ist erfindungsgemäß die grundlegende Geometrie des Mikrofongehäuses so gewählt, daß dieses Gehäuse prinzipiell aus zwei Schenkeln besteht, die in einem Winkel zueinander angeordnet sind, wobei der eine Schenkel, der vorzugsweise zylinderförmig ausgeführt ist, die Mikrofonkapsel sowie die schallaufnehmende, vorzugsweise kreisförmige Membran enthält und der andere Schenkel die elektrische Durchführungsanordnung und vorzugsweise elektronische Bauelemente aufnimmt, wie im folgenden näher erläutert wird.

Weitere bevorzugte Ausführungsformen der Erfindung ergeben sich aus den Unteransprüchen.

Insbesondere ist aus den bereits geschilderten Gründen die Geometrie des Mikrofongehäuses bevorzugt so gewählt, daß bei Implantation des Mikrofons in der Mastoidhöhle der Schenkel, der die Schalleintrittsmembran enthält, vom Mastoid aus in eine artifizielle Bohrung in der hinteren, knöchernen Gehörgangswand hineinragt und die Schalleintrittsmembran die Haut der Gehörgangswand berührt, und daß der Schenkel des Mikrofongehäuses, der die elektrische Durchführungsanordnung enthält, im Bereich der Mastoidspitze plaziert ist. Eine über eine vollständige Mastoidektomie hinausgehende Erweiterung der Mastoidhöhle ist hierfür nicht erforderlich.

Da die operative Plazierung des beschriebenen Mikrofonmoduls so erfolgt, daß das Ende des Gehäuseschenkels, der die Durchführungsanordnung enthält, in Richtung der Mastoidspitze weist, ist es zweckmäßig, wenn eine an die Durchführungsanordnung angeschlossene elektrische Leitung im Anschlußbereich in einem rechten Winkel zu dem Gehäuseschenkel steht, der die elektrische Durchführungsanordnung enthält. Dadurch wird ein scharfer Knick bzw. ein sehr kleiner Biegeradius der Leitung vermieden, der andernfalls durch die Anatomie der eröffneten Mastoidhöhle im Bereich der Mastoidspitze vorgegeben wäre, und der Leitungsanschluß an das Mikrofonmodul ist somit bezüglich mechanischer Dauerbeanspruchungen entlastet.

Die elektrische Durchführungsanordnung kann mehrpolig ausgeführt sein. Bei zweipoliger Ausführung ist die Anordnung vorzugsweise so getroffen, daß sie unter Verwendung des elektrischen Prinzips der Phantomspeisung arbeitet, d.h. daß ein Pol gemeinsam das Nutzsignalpotential und das energieversorgende Gleichspannungspotential zur Energieversorgung des Mikrofons führt. Ist die elektrische Durchführungsanordnung einpolig ausgeführt, so kann dieser eine Pol gemeinsam das Nutzsignalpotential und das energieversorgende Gleichspannungspotential führen, während ein zweiter Pol einer zuführenden Leitung an das Mikrofongehäuse angeschlossen ist, das für diesen Zweck elektrisch leitend ausgeführt ist und durch interne Verbindung mit der Mikrofonkaspel-Beschaltung das Massepotential führt.

Unabhängig von der Realisierung der internen Schallwandlung in ein elektrisches Signal sind die zum elektrischen Anschluß der internen Mikrofonkapsel nach dem Prinzip der Phantomspeisung benötigten elektronische Bauelemente und Komponenten vorzugsweise ebenfalls in dem Mikrofongehäuse, insbesondere in dem Gehäuse-Schenkel, der die elektrische Durchführungsanordnung aufnimmt, untergebracht. Ferner können in dem Mikrofongehäuse elektronische Bauelemente untergebracht sein, die eine elektrische Impedanzwandlung und/oder eine elektrische Signalverstärkung und/oder Schutzmaßnahmen gegen elektrische, magnetische und/oder elektromagnetische Umwelteinflüsse ermöglichen. Diese letztgenannten Maßnahmen schließen in der Regel Hochfrequenzkondensatoren und Varistoren ein, um einerseits das Mikrofonmodul selbst gegen Zerstörung aufgrund energiereicher elektromagnetischer Einwirkung zu schützen, und um andererseits kapazitive und induktive Einstreuungen in den Signalpfad zu minimieren bzw. zu unterdrücken, die zu Störungen in der weiteren Audiosignalverarbeitung des an das Mikrofonmodul angeschlossenen Implantatsystems führen können.

Das Mikrofongehäuse einschließlich der schallaufnehmenden Membran und der elektrischen Durchführungseinrichtung ist vorzugsweise mit biokompatiblen Metallen, beispielhaft aus Reintitan oder biokompatiblen Titan-Legierungen, ausgeführt und allseitig hermetisch gasdicht verschlossen.

Die in dem Mikrofongehäuse untergebrachte Mikrofonkapsel ist insbesondere zum Arbeiten nach dem elektrodynamischen, elektromagnetischen, dielektrischen und vorzugsweise nach dem Elektret-Prinzip ausgelegt. Insbesondere kann es sich um eine Elektret-Miniaturausführung mit integriertem Feldeffekt-Transistor zur elektrischen Impedanzwandlung handeln. Das Mikrofon kann auch entsprechend dem Kondensator-Mikrofon-Prinzip in NF- bzw. HF-Schaltung ausgeführt sein. Insbesondere kann die Mikrofonfunktion durch ein Halbleiter-Mikrofon in Siliziumtechnologie realisiert sein, das mit Methodiken der Mikrosystemtechnik aufgebaut ist.

Um die das Mikrofonmodul mit dem Hörhilfe-Implantatsystem verbindende elektrische Anschlußleitung weitgehend unempfindlich gegenüber elektromagnetischen Umwelteinflüssen (EMV) zu machen, ist diese Anschlußleitung vorzugsweise als Twisted-Pair- oder Koaxial-Leitung ausgeführt.

Das hier beschriebene implantierbare Mikrofon kann Bestandteil von teil- oder vollimplantierbaren Hörhilfen wie z.B. Cochlea Implantaten mit elektrischer Reizung der Hörbahn, Hörgeräten mit mechanischer Stimulation des Mittelohres oder des Innenohres sowie aktiven Maskierersystemen zur Tinnitus-Behandlung sein, die mit adäquaten elektronischen Signalverarbeitungs- und Energieversorgungssystemen und Stimulationsmitteln die Rehabilitation eines teilweisen bzw. vollständigen Ausfalls oder die apparative Behandlung der Beeinträchtigung eines oder beider Hörorgane ermöglichen.

Unter dem Oberbegriff Hörhilfen sind hier generell teil- oder vollimplantierbare Systeme zu verstehen, die mit Hilfe des beschriebenen Mikrofonmoduls, adäquaten elektronischen Signalverarbeitungs- und Energieversorgungssystemen und Stimulationsmitteln die Rehabilitation eines teilweisen bzw. vollständigen Ausfalls oder die apparative Behandlung der Beeinträchtigung eines oder beider Hörorgane ermöglichen. Hierunter sind insbesondere Cochlea Implantate mit elektrischer Reizung der Hörbahn, implantierbare Hörgeräte mit mechanischer Stimulation des Mittelohres oder des Innenohres sowie aktive, implantierbare Maskierersysteme zur Tinnitus-Behandlung zu verstehen.

Bevorzugte Ausführungsformen des implantierbaren Mikrofonmoduls werden nachstehend an Hand der Zeichnungen näher erläutert. Es zeigen:
- **Fig. 1**: einen Querschnitt durch das menschliche Gehör mit implantiertem Mikrofonmodul;
- **Fig. 2**: eine Aufsicht in die Mastoidhöhle nach erfolgter Mastoidektomie mit implantiertem Mikrofonmodul;
- **Fig. 3**: eine Anwendung des implantierbaren Mikrofonmoduls bei einem vollständig implantierbaren Hörgerät mit elektromechanischer Stimulation der Ossikelkette; und
- **Fig. 4**: eine Anwendung des implantierbaren Mikrofonmoduls bei einem vollständig implantierbaren Cochlea Implantat mit direkter elektrischer Stimulation der Cochlea.

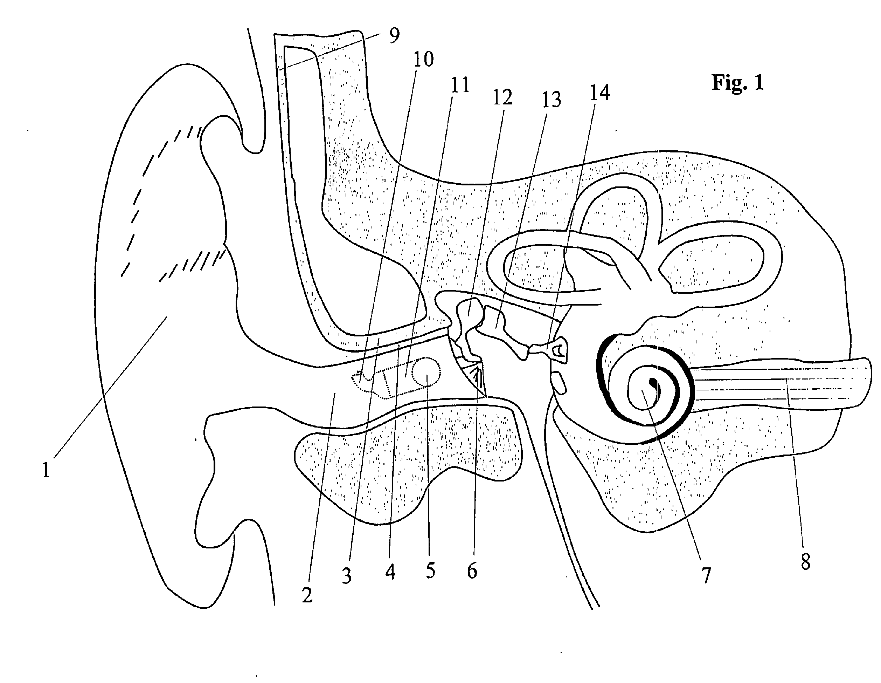

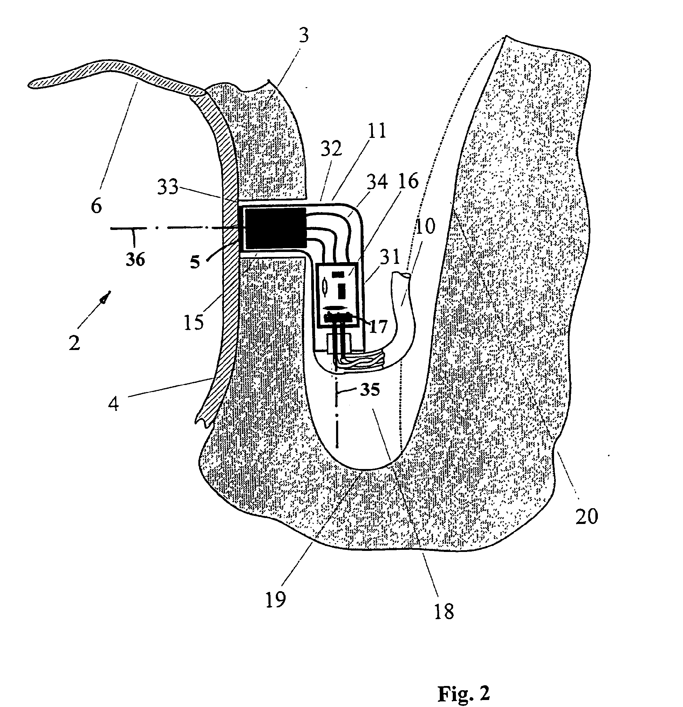

**Fig. 1** zeigt einen Querschnitt durch das menschliche Gehör mit dem Außenohr **1**, dem Gehörgang **2**, der knöchernen Gehörgangswand **3**, der Haut des Gehörgangs **4**, dem Trommelfell **6**, der Gehörknöchelchenkette bestehend aus Hammer **12**, Amboß **13** und Steigbügel **14**, dem Innenohr (Cochlea) **7** und dem Hörnerv **8**. Ein im Bereich der knöchernen hinteren Gehörgangswand vom Mastoid **9** aus eingebrachtes, ein Mikrofongehäuse **11** aufweisendes Mikrofonmodul ist etwa maßstabsgetreu im implantierten Zustand eingezeichnet, wobei die Gehörgangswandhaut **4** eine Membran **5** berührt, welche einen Teil des Mikrofongehäuses **11** bildet. Ein eine elektrische Durchführungsanordnung **17** mit angeschlossener Leitung **10** beinhaltender erster Schenkel **31** des Mikrofongehäuses **11**, dessen Achse in **Fig. 2** schematisch bei **35** angedeutet ist, weist im implantierten Zustand etwa in Richtung der Mastoidspitze. Die elektrische Durchführungsanordnung **17** befindet sich in einem Bereich des Schenkels **31**, der außerhalb einer zu der Schalleintrittsmembran **5** senkrechten Projektion der Mikrofonkapsel **15** liegt.

In **Fig. 2** ist das Mikrofongehäuse **11** schematisch im implantierten Zustand im Querschnitt als Aufsicht auf die eröffnete Mastoidhöhle **18** nach Mastoidektomie dargestellt. Das Mikrofongehäuse **11** ist mit einem eine Mikrofonkapsel **15** aufnehmenden zweiten Gehäuseschenkel **32** so in eine entsprechende artifizielle Bohrung **33** in der knöchernen Gehörgangswand **3** eingeführt, daß die Haut **4** des Gehörgangs die kreisförmige Membran **5** mechanisch sicher und auf der ganzen Kreisfläche der Membran berührt. Der Gehäuseschenkel **32** hat eine Achse **36** (**Fig. 2**), die in der veranschaulichten Ausführungsform im wesentlichen in einem rechten Winkel zu der Achse **35** des Gehäuseschenkels **31** steht. Der Bereich der Gehörgangswandhaut, der die Membran berührt, stellt zusammen mit der Membran ein mechanisch schwingfähiges Gebilde dar, das durch eine in den äußeren Gehörgang **2** mit dem Trommelfell **6** einfallende Schallwelle in mechanische Schwingungen versetzt wird, die sich als akustische Druckschwankungen im hermetisch abgeschlossenen inneren Volumen des Mikrofongehäuses **11** abbilden. Diese Druckschwankungen werden durch die interne Mikrofonkapsel **15** in ein elektrisches Signal umgewandelt, das einer Signalverarbeitungseinheit **16** über interne elektrische Leitungen **34** zugeführt wird.

Die Signalverarbeitungseinheit **16** kann Komponenten zur elektrischen Phantomspeisung des Systems, aktiv verstärkende Elemente, impedanzwandelnde Bauelemente und Komponenten zur Unterdrückung bzw. Dämpfung von elektrischen, magnetischen und/oder elektro-magnetischen Umwelteinflüssen beinhalten. Die Signalverarbeitungseinheit **16** ist ausgangsseitig elektrisch an die Durchführungsanordnung **17** angeschlossen, die hermetisch dicht in das Mikrofongehäuse **11** eingebracht ist. Die Signalverarbeitungseinheit **16** ist zusammen mit der Durchführungsanordnung **17** in dem Schenkel **31** des Mikrofongehäuses **11** untergebracht, der unter einem Winkel, hier vorzugsweise als rechter Winkel dargestellt, zu dem Schenkel **32** des Mikrofongehäuses steht, der mit der schallaufnehmenden Membran **5** versehen ist und die interne Mikrofonkapsel **15** enthält. Die elektrische Leitung **10** ist vorzugsweise im rechten Winkel so an die elektrische Durchführungsanordnung **17** angeschlossen, daß die Leitung **10** knickfrei und unter Vermeidung sehr kleiner Biegeradien aus der Mastoidspitze **19** herausgeführt werden kann. Die elektrische Durchführungsanordnung **17** und die Leitung **10** sind hier für den vorzugsweisen Fall einer energetisehen Versorgung des Mikrofonmoduls über eine Phantomspeisung, bei der die Nutzsignal-Wechselspannung und die versorgende Gleichspannung gemeinsam über einen Pol und das Massesignal über einen zweiten Pol geführt werden, zweipolig ausgeführt. In diesem Fall ist die Leitungsanordnung der elektrischen Implantatleitung **10** vorzugsweise nach dem Twisted-Pair-Prinzip ausgeführt, um elektromagnetische Umwelteinflüsse auf die Implantatleitung **10** zu minimieren.

Aus **Fig. 2** sind die beengten Raumverhältnisse in der eröffneten Mastoidhöhle ersichtlich, die wesentlich durch den Abstand zwischen hinterer Gehörgangswand **3** und dem knöchernen Wall des Sinus Sigmoideus **20**, der eine wichtige venöse Versorgung enthält und daher chirurgisch nicht abgetragen werden kann, gegeben sind. Erst die Unterbringung der beschriebenen Komponenten in einem zweischenkeligen Gehäuse erlaubt die Wahl des dargestellten Implantationsortes. Eine axiale Anordnung aller Mikrofon-Modul-Komponenten einschließlich des elektrischen Leitungsanschlusses ist nicht möglich. Weiterhin ist aus **Fig. 2** ersichtlich, daß der die Durchführungsanordnung **17** enthaltende Schenkel **31** des Mikrofongehäuses **11** gegenüber der Ebene der kreisförmigen Gehäusemembran **5** mindestens um die Dicke der knöchernen Gehörgangswand **3** zurückgesetzt sein muß, um ein so tiefes Einführen des Gehäuses **11** in die artifizielle Bohrung **33** in der Gehörgangswand zu gewährleisten, daß die Membran **5** des Mikrofon-gehäuses die Haut **4** der Gehörgangswand sicher in allen Membranbereichen berührt.

Aus **Fig. 1** und **Fig. 2** ist ersichtlich, daß das Mikrofongehäuse **11** dann mit dem Schenkel **31**, der die Durchführungsanordnung 17 mit angeschlossener Leitung **10** enthält, sinnvollerweise in Richtung Mastoidspitze **19** orientiert plaziert werden sollte, wenn im Falle eines vollimplantierbaren elektromechanischen Hörgeräts Komponenten des elektromechanischen Wandlers in der Mastoidhöhle, dem Antrum oder direkt im Mittelohrbereich implantiert werden.

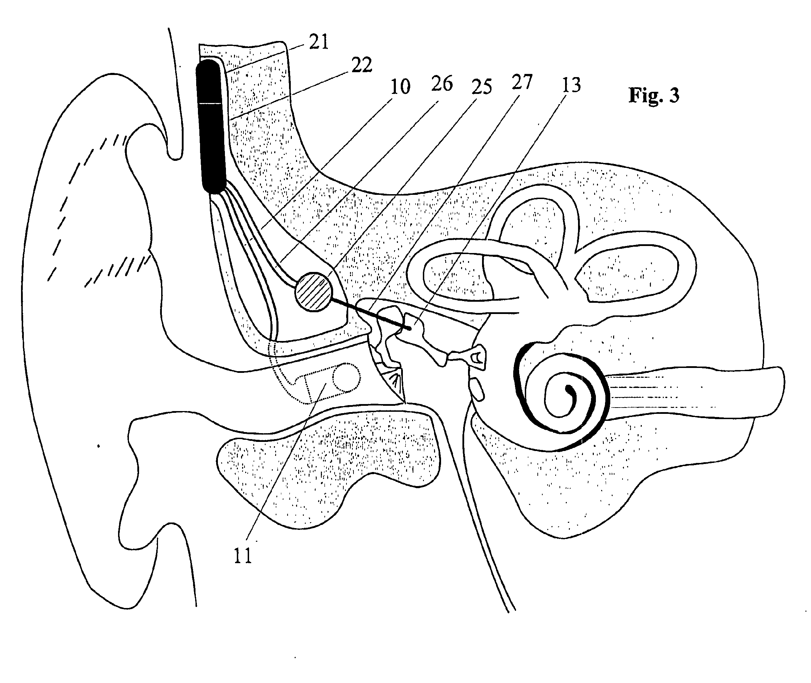

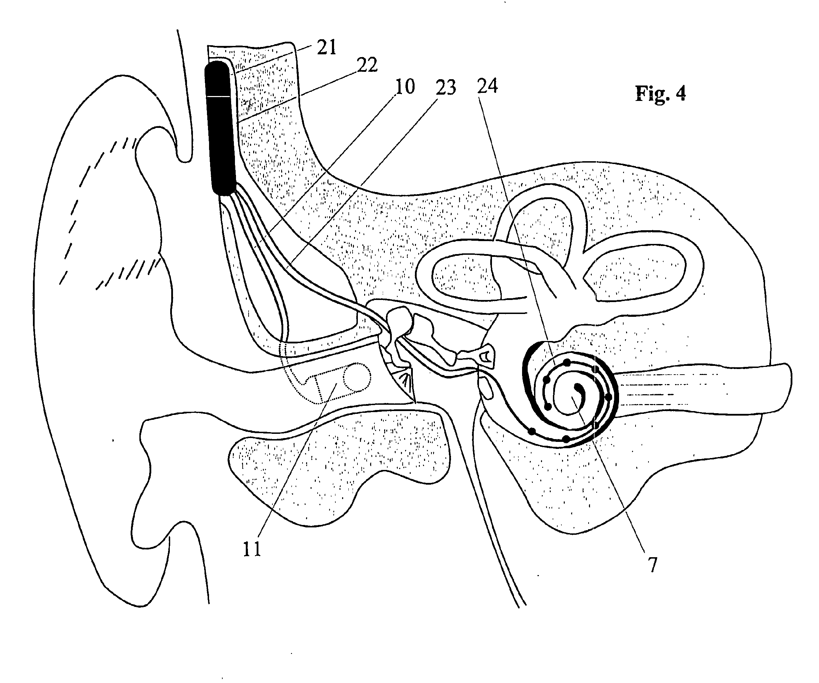

Eine andere Orientierung des Mikrofongehäuses, d.h. ein Freiheitsgrad der operativen Plazierung des Mikrofonmoduls, kann dann gegeben sein, wenn im Falle einer vollständig implantierbaren Hörhilfe mit direkter elektrischer Stimulation der Cochlea nur das entsprechende Reizelektroden-Array vom Mastoid aus in Richtung der Cochlea geführt werden muß und somit die oben beschriebenen stark eingeschränkten Platz- und Volumenverhältnisse nicht gegeben sind. Solche Ausführungskombinationen mit dem beschriebenen Mikrofonmodul sind in **Fig. 3** und **Fig. 4** schematisch dargestellt.

In **Fig. 3** ist ein vollständig implantierbares Hörgerät mit elektromechanischer Stimulation der Ossikelkette unter Verwendung eines in dem Gehäuse **11** untergebrachten Mikrofonmoduls dargestellt, wobei eine über die Leitung **10** mit dem Mikrofonmodul in Verbindung stehende signalverarbeitende Einheit **21**, die auch die elektrische Energieversorgung sowie Komponenten zur drahtlosen Kommunikation mit der Außenwelt enthalten kann, in einem ausgefrästen Knochenbett **22** im Mastoidbereich untergebracht ist. Eine das elektrische Ausgangssignal führende Implantatleitung **26** führt zu einem elektromechanischen Wandler **25**, der beispielhaft in der Mastoidhöhle oder im Antrum plaziert ist. Die mechanischen Schwingungen des Wandlers **25** werden über ein adäquates mechanisches Koppelelement **27** der Ossikelkette zugeführt, im dargestellten Beispiel an den Amboß **13**.

In **Fig. 4** ist eine vollständig implantiert ausgeführte Hörhilfe mit direkter elektrischer Stimulation des Gehörs über ein in die Cochlea **7** eingeführtes Reizelektrodenarray **24** unter Verwendung des in dem Gehäuse **11** befindlichen Mikrofonmoduls dargestellt. Auch hier ist die Signalverarbeitungseinheit **21** mit allen für einen transkutanen Implantatbetrieb notwendigen Komponenten wie in **Fig. 3** in dem artifiziellen Knochenbett **22** im Mastoid plaziert, wobei eine elektrische Zuleitung **23** zu dem Reizelektrodenarray **24** vom Mastoid durch den Mittelohrbereich zur Cochlea **7** führt.

## Patentansprüche

1. Implantierbares Mikrofon für eine der Anregung des Gehörs dienende implantierbare Hörhilfe mit einer Mikrofonkapsel (15), die in einem Gehäuse (11) allseitig hermetisch dicht untergebracht ist, und mit einer elektrischen Durchführungsanordnung (17) zum Durchführen mindestens eines elektrischen Anschlusses von dem Innenraum des Gehäuses zu dessen Außenseite, wobei das Gehäuse mindestens zwei Schenkel (31, 32) aufweist, deren Achsen (35, 36) in einem Winkel mit Bezug aufeinander ausgerichtet sind, wobei der eine Schenkel (32) die Mikrofonkapsel aufnimmt und mit einer Schalleintrittsmembran (5) versehen ist, und wobei der andere Schenkel (31) gegenüber der Ebene der Schalleintrittsmembran zurückversetzt ist und die elektrische Durchführungsanordnung (17) in einem Bereich aufnimmt, der außerhalb einer zur Schalleintrittsmembran senkrechten Projektion der Mikrofonkapsel liegt.

2. Implantierbares Mikrofon nach Anspruch 1, **dadurch gekennzeichnet, daß** der Winkel der Achsen (35, 36) der beiden Schenkel (31, 32) des Mikrofongehäuses (11) zueinander näherungsweise ein rechter Winkel ist.

3. Implantierbares Mikrofon nach einem der vorhergehenden Ansprüche, **dadurch gekennzeichnet, daß** die Geometrie des Mikrofongehäuses (11) so gewählt ist, daß bei Implantation des Mikrofons in der Mastoidhöhle (18) der Schenkel (32), der die Schalleintrittsmembran (5) enthält, vom Mastoid (9) aus in eine artifizielle Bohrung (33) in der hinteren, knöchernen Gehörgangswand (3) hineinragt und die Schalleintrittsmembran die Haut (4) der Gehörgangswand berührt, und daß der Schenkel (31) des Mikrofongehäuses, der die elektrische Durchführungsanordnung (17) enthält, im Bereich der Mastoidspitze (19) plaziert ist.

4. Implantierbares Mikrofon nach einem der vorhergehenden Ansprüche, **dadurch gekennzeichnet, daß** ferner an die Durchführungsanordnung (17) eine elektrische Leitung (10) angeschlossen ist, die im Anschlußbereich in einem rechten Winkel zu dem Gehäuseschenkel (31) steht, der die elektrische Durchführungsanordnung enthält.

5. Implantierbares Mikrofon nach einem der vorhergehenden Ansprüche, **dadurch gekennzeichnet, daß** die elektrische Durchführungsanordnung (17) mehrpolig ausgeführt ist.

6. Implantierbares Mikrofon nach einem der vorhergehenden Ansprüche, **dadurch gekennzeichnet, daß** die elektrische Durchführungsanordnung (17) zweipolig unter Verwendung des elektrischen Prinzips der Phantomspeisung ausgeführt ist.

7. Implantierbares Mikrofon nach einem der Ansprüche 1 bis 4, **dadurch gekennzeichnet, daß** die elektrische Durchführungsanordnung (17) einpolig ausgeführt ist und dieser eine Pol gemeinsam das Nutzsignalpotential und das energieversorgende Gleichspannungspotential führt, daß das Mikrofongehäuse (11) elektrisch leitend ausgeführt ist, und daß ein zweiter Pol einer zuführenden Leitung (10) angeschlossen ist und das Massepotential über das Mikrofongehäuse geführt ist.

8. Implantierbares Mikrofon nach einem der vorhergehenden Ansprüche, **dadurch gekennzeichnet, daß** in dem Mikrofongehäuse (11) ferner elektronische Bauelemente (16) untergebracht sind, die einen elektrischen Anschluß der internen Mikrofonkapsel (15) nach dem Prinzip der Phantomspeisung ermöglichen.

9. Implantierbares Mikrofon nach einem der vorhergehenden Ansprüche, **dadurch gekennzeichnet, daß** in dem Mikrofongehäuse (11) ferner elektronische Bauelemente (16) untergebracht sind, die eine Minimierung elektrischer, magnetischer und/oder elektromagnetischer Umwelteinflüsse gewährleisten.

10. Implantierbares Mikrofon nach einem der vorhergehenden Ansprüche, **dadurch gekennzeichnet, daß** in dem Mikrofongehäuse (11) ferner elektronische Bauelemente (16) untergebracht sind, die eine elektrische Verstärkung und/oder eine Impedanzwandlung des Mikrofonsignals realisieren.

11. Implantierbares Mikrofon nach einem der vorhergehenden Ansprüche, **dadurch gekennzeichnet, daß** das Mikrofongehäuse (11) einschließlich der Schalleintrittsmembran (5) in Reintitan oder biokompatiblen Titan-Legierungen ausgeführt ist.

12. Implantierbares Mikrofon nach einem der vorhergehenden Ansprüche, **dadurch gekennzeichnet, daß** die Mikrofonkapsel (15) zum Arbeiten nach dem elektrodynamischen, elektromagnetischen, dielektrischen, piezoelektrischen oder vorzugsweise nach dem Elektret-Prinzip ausgelegt ist.

13. Implantierbares Mikrofon nach einem der vorhergehenden Ansprüche, **dadurch gekennzeichnet, daß** das Mikrofongehäuse (11) ein internes Halbleiter-Mikrofon (15) enthält, das nach Methoden der Mikrosystemtechnik gefertigt ist.

14. Implantierbares Mikrofon nach einem der vorhergehenden Ansprüche, **dadurch gekennzeichnet, daß** die externe, elektrische Anschlußleitung (10) als Twisted-Pair- oder Koaxial-Leitung ausgeführt ist.

15. Implantierbares Mikrofon nach einem der vorhergehenden Ansprüche, **dadurch gekennzeichnet, daß** es Bestandteil von teil- oder vollimplantierbaren Hörhilfen wie z.B. Cochlea Implantaten mit elektrischer Reizung der Hörbahn, Hörgeräten mit mechanischer Stimulation des Mittelohres oder des Innenohres sowie aktiven Maskierersystemen zur Tinnitus-Behandlung ist, die mit adäquaten elektronischen Signalverarbeitungs- und Energieversorgungssystemen (21) und Stimulationsmitteln (25, 27) die Rehabilitation eines teilweisen bzw. vollständigen Ausfalls oder die apparative Behandlung der Beeinträchtigung eines oder beider Hörorgane ermöglichen.

## Claims

1. Implantable microphone for an implantable hearing aid used to stimulate the hearing, comprising a microphone capsule (15) disposed in a housing (11) in an all-side hermetically sealed manner, and an electrical feedthrough arrangement (17) for feeding through at least one electrical connector from the interior of the housing to the exterior thereof, wherein the housing includes at least two legs (31, 32) the axes (35, 36) of which are disposed at an angle to each other, wherein one (32) of the legs accommodates the microphone capsule and is provided with a sound inlet diaphragm (5), and wherein the other one (31) of the legs is set back relative to the plane of the sound inlet diaphragm and accommodates the electrical feedthrough arrangement in a region which is outside of a projection of the microphone capsule that is normal to the sound inlet diaphragm.

2. Implantable microphone as claimed in claim1, **characterized in that** the relative angle defined by the axes (35, 36) of the two legs (31, 32) of the microphone housing (11) is approximately a right angle.

3. Implantable microphone as claimed in any one of the preceding claims, **characterized in that** the geometry of the microphone housing (11) is selected such upon implantation of the microphone in the mastoid cavity the leg (32) comprising the sound inlet diaphragm (5) extends from the mastoid (9) into an artificial bore (33) in the osseous posterior auditory canal wall (3) and the sound inlet diaphragm contacts the skin (4) of the auditory canal wall, and that the leg (31) of the microphone housing containing the electrical feedthrough arrangement (17) is disposed in the region of the mastoid tip (19).

4. Implantable microphone as claimed in any one of the preceding claims, **characterized in that** the electrical feedthrough arrangement (17) has attached thereto an electrical conduit (10) which, in the connection region, extends at a right angle to the housing leg (31) containing the electrical feedthrough arrangement.

5. Implantable microphone as claimed in any one of the preceding claims, **characterized in that** the electrical feedthrough arrangement (17) is multipolar.

6. Implantable microphone as claimed in any one of the preceding claims, **characterized in that** the electrical feedthrough arrangement (17) is bipolar making use of the electrical principal of phantom feed-in.

7. Implantable microphone as claimed in any one of claims 1 to 4, **characterized in that** the electrical feedthrough arrangement (17) is unipolar and that this single pole is carrying both the useful signal potential and the energy supply DC potential, that the microphone housing (11) is electrically conductive, and that a second pole of a supply conduit (10) is connected to carry the ground potential via the microphone housing.

8. Implantable microphone as claimed in any one of the preceding claims, **characterized in that** electronic components (16) permitting an electrical connection of the microphone capsule (15) in conformity with the principal of phantom feed-in also are accommodated in the microphone housing (11).

9. Implantable microphone as claimed in any one of the preceding claims, **characterized in that** electronic components (16) providing for a minimization of electrical, magnetic and/or electromagnetic environmental influences also are accommodated in the microphone housing (11).

10. Implantable microphone as claimed in any one of the preceding claims, **characterized in that** electronic components (16) providing for an electrical amplification and/or impedance transformation of the microphone signal also are accommodated in the microphone housing (11).

11. Implantable microphone as claimed in any one of the preceding claims, **characterized in that** the microphone housing (11), including the sound inlet diaphragm (5), is made of pure titanium or of biocompatible titanium alloys.

12. Implantable microphone as claimed in any one of the preceding claims, **characterized in that** the microphone capsule (15) is designed for operation in conformity with the electrodynamic, electromagnetic, dielectric, piezoelectric or, preferably, the electret principal.

13. Implantable microphone as claimed in any one of the preceding claims, **characterized in that** the microphone housing (11) contains an internal semiconductor microphone (15) that is produced in conformity with methods of the microsystem technique.

14. Implantable microphone as claimed in any one of the preceding claims, **characterized in that** the external electrical connector conduit (10) is a twisted pair coaxial conduit.

15. Implantable microphone as claimed in any one of the preceding claims, **characterized in that** it is part of partially or fully implantable hearing aids, such as cochlea implants providing for electrical stimulation of the auditory pathway, hearing aids providing for mechanical stimulation of the middle ear or the inner ear, or of masking systems for tinnitus treatment, permitting, with adequate electronic signal processing and energy supply systems (21) and stimulation means (25, 27), the rehabilitation of a partial or complete failure or the apparative treatment of an disturbance of one or both acoustic organs.

## Revendications

1. Microphone implantable pour une prothèse auditive implantable servant à l'excitation de l'ouïe comprenant une capsule à microphone (15) qui est logée de façon hermétiquement étanche de tous côtés dans un boîtier (11), et un agencement de passage (17) électrique pour le passage d'au moins un branchement électrique de l'espace intérieur du boîtier vers son côté extérieur, le boîtier présentant au moins deux branches (31, 32) dont les axes (35, 36) sont orientés en formant un angle les uns par rapport aux autres, l'une des branches (32) présentant la capsule à microphone et étant dotée d'une membrane d'entrée de son (5), et l'autre branche (31) étant en retrait par rapport au plan de la membrane d'entrée de son et recevant l'agencement de passage (17) électrique dans une zone qui se situe à l'extérieur d'une projection, perpendiculaire à la membrane d'entrée de son, de la capsule à microphone.

2. Microphone implantable selon la revendication 1, **caractérisé en ce que** les angles des axes (35, 36) des deux branches (31, 32) du boîtier du microphone (11) forment approximativement un angle droit les uns par rapport aux autres.

3. Microphone implantable selon l'une des revendications précédentes, **caractérisé en ce que** la géométrie du boîtier du microphone (11) est choisie de telle sorte que, en cas d'implantation du microphone dans la cavité de mastoïde (18), la branche (32), qui contient la membrane d'entrée de son (5), dépasse du mastoïde (9) dans un percement (33) artificiel dans la paroi arrière et osseuse du conduit auditif (3) et la membrane d'entrée de son touche la peau (4) de la paroi du conduit auditif, et **en ce que** la branche (31) du boîtier du microphone, qui contient l'agencement de passage (17) électrique, est placée dans la zone de la pointe du mastoïde (19).

4. Microphone implantable selon l'une des revendications précédentes, **caractérisé en ce qu'**à l'agencement de passage (17) est raccordée également une ligne (10) électrique, qui forme dans la zone de raccordement un angle droit par rapport à la branche de boîtier (31) qui contient l'agencement de passage électrique.

5. Microphone implantable selon l'une des revendications précédentes, **caractérisé en ce que** l'agencement de passage (17) électrique est réalisé avec plusieurs pôles.

6. Microphone implantable selon l'une des revendications précédentes, **caractérisé en ce que** l'agencement de passage (17) électrique est réalisé avec deux pôles en utilisant le principe électrique de l'alimentation en fantôme.

7. Microphone implantable selon l'une des revendications 1 à 4, **caractérisé en ce que** l'agencement de passage (17) électrique est réalisé avec un pôle et ce pôle unique guide conjointement le potentiel de signal utile et le potentiel de tension continue alimentant en énergie, **en ce que** le boîtier de microphone (11) est réalisé de façon électroconductrice, et **en ce qu'**un second pôle d'une ligne (10) d'alimentation est raccordé et le potentiel de masse est guidé par le boîtier de microphone.

8. Microphone implantable selon l'une des revendications précédentes, **caractérisé en ce que** dans le boîtier de microphone (11) sont logés également des composants (16) électroniques qui permettent un raccordement électrique de la capsule de microphone (15) interne selon le principe de l'alimentation en fantôme.

9. Microphone implantable selon l'une des revendications précédentes, **caractérisé en ce que** dans le boîtier de microphone (11) sont logés également des composants (16) électroniques qui garantissent une minimisation d'influences électriques, magnétiques et/ou électromagnétiques de l'environnement.

10. Microphone implantable selon l'une des revendications précédentes, **caractérisé en ce que** dans le boîtier du microphone (11) sont logés également des composants (16) électroniques qui réalisent une amplification électrique et/ou une conversion d'impédance du signal de microphone.

11. Microphone implantable selon l'une des revendications précédentes, **caractérisé en ce que** le boîtier de microphone (11), y compris la membrane d'entrée de son (5), est réalisé en titane pur ou dans des alliages de titane biocompatibles.

12. Microphone implantable selon l'une des revendications précédentes, **caractérisé en ce que** la capsule de microphone (15) est conçue pour travailler selon le principe électrodynamique, électromagnétique, diélectrique, piézoélectrique ou de préférence selon le principe d'électret.

13. Microphone implantable selon l'une des revendications précédentes, **caractérisé en ce que** le boîtier de microphone (11) contient un microphone à semi-conducteurs (15) interne qui est fabriqué selon des méthodes de la technique de microsystème.

14. Microphone implantable selon l'une des revendications précédentes, **caractérisé en ce que** la ligne de raccordement (10) électrique externe est réalisée sous forme de ligne à paire torsadée ou de ligne coaxiale.

15. Microphone implantable selon l'une des revendications précédentes, **caractérisé en ce qu'**il fait partie de prothèses auditives partiellement ou complètement implantables telles que des implants cochléaires avec stimulation électrique de la voie acoustique, de prothèses auditives avec stimulation mécanique de l'oreille moyenne ou de l'oreille interne ainsi que de systèmes masqueurs actifs pour le traitement de bourdonnement, qui permettent, au moyen de systèmes de traitement de signal et d'alimentation en énergie (21) électroniques adéquats et de moyens de stimulation (25, 27) la rééducation d'une défaillance partielle ou complète ou le traitement avec appareillage de l'altération d'un ou des deux organes auriculaires.
